(19) Europäisches Patentamt
European Patent Office
Office européen des brevets

(11) EP 2 683 475 B1

(12) **FASCICULE DE BREVET EUROPEEN**

(45) Date de publication et mention de la délivrance du brevet:
**19.06.2019 Bulletin 2019/25**

(21) Numéro de dépôt: **12707331.0**

(22) Date de dépôt: **07.03.2012**

(51) Int Cl.:
***B01J 13/10*** *(2006.01)*
***A61K 8/11*** *(2006.01)*
***A61K 9/50*** *(2006.01)*
***C11D 3/50*** *(2006.01)*

(86) Numéro de dépôt international:
**PCT/EP2012/053917**

(87) Numéro de publication internationale:
**WO 2012/120043 (13.09.2012 Gazette 2012/37)**

(54) **PROCÉDÉ DE FORMATION DE GOUTTES D'UNE PREMIÈRE PHASE DISPERSÉES DANS UNE DEUXIÈME PHASE SENSIBLEMENT IMMISCIBLE AVEC LA PREMIÈRE PHASE**

VERFAHREN ZUR TROPFENBILDUNG EINER DISPERSION EINER ERSTEN PHASE IN EINER WEITGEHEND MIT DER ERSTEN PHASE UNMISCHBAREN ZWEITEN PHASE

METHOD FOR FORMING DROPS OF A FIRST PHASE DISPERSED IN A SECOND PHASE SUBSTANTIALLY IMMISCIBLE WITH THE FIRST PHASE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.03.2011 FR 1151885**
**08.03.2011 FR 1151882**

(43) Date de publication de la demande:
**15.01.2014 Bulletin 2014/03**

(60) Demande divisionnaire:
**19171412.0**

(73) Titulaire: **Capsum**
**13013 Marseille (FR)**

(72) Inventeurs:
- **GOUTAYER, Mathieu**
  **F-35400 Saint-Malo (FR)**
- **BIBETTE, Jérôme**
  **F-75005 Paris (FR)**

(74) Mandataire: **Lavoix**
**2, place d'Estienne d'Orves**
**75441 Paris Cedex 09 (FR)**

(56) Documents cités:
**EP-A1- 1 524 030**
**FR-A1- 2 939 012**
**GB-A- 2 135 954**
**US-A1- 2007 145 326**

- **WEN S ET AL: "Microcapsules through polymer complexation - Part 3: encapsulation and culture of human Burkitt lymphoma cells in vitro", BIOMATERIALS, ELSEVIER SCIENCE PUBLISHERS BV., BARKING, GB, vol. 16, no. 4, 1 janvier 1995 (1995-01-01), pages 325-335, XP004033057, ISSN: 0142-9612, DOI: 10.1016/0142-9612(95)93261-B**
- **DATABASE WPI Week 201012 Thomson Scientific, London, GB; AN 2010-A47048 XP002662640, -& CN 101 612 139 A (UNIV SICHUAN) 30 décembre 2009 (2009-12-30)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 683 475 B1

## Description

La présente invention concerne un procédé de formation de gouttes d'une première phase dispersées dans une deuxième phase sensiblement immiscible avec la première phase, chaque goutte comportant un coeur formé de première phase et une écorce formée d'une couche de coacervat interposée entre la première phase et la deuxième phase, selon le préambule de la revendication 1.

Un tel procédé est destiné par exemple à former une dispersion de type eau dans l'huile comprenant des gouttes stables de phase dispersée, de taille supérieure à 500 microns, et notamment comprise entre 500 microns et 2500 microns, de préférence entre 700 microns et 1500 microns. En variante, la dispersion est de type huile dans eau.

EP 1524030 A1 décrit un procédé du type précité.

La phase dispersée est destinée à contenir par exemple un produit cosmétique, un produit biologiquement actif, ou un produit comestible propre à être consommé.

On connaît de WO 03/002248 un procédé du type précité, destiné à disperser sous forme de gouttes un détergent dans une phase huileuse. L'écorce des gouttes est formée par un coacervat de polymères.

A cet égard, un premier précurseur du coacervat, formé par de la mélamine, est mélangé avec un deuxième précurseur du coacervat, formé par du formaldéhyde. Ces polymères sont placés dans une solution aqueuse contenant le détergent. Un catalyseur de coacervation, constitué par de l'acide citrique, est également ajouté dans la phase aqueuse.

Cette phase aqueuse est ensuite dispersée dans un solvant huileux en présence d'un polymère de stabilisation amphiphatique.

Puis, la dispersion est chauffée à une température relativement élevée pour engendrer la formation du coacervat à l'interphase, par séparation de phase et polymérisation.

Un tel procédé ne donne pas entière satisfaction. En effet, bien que la présence du stabilisant dans la phase huileuse limite la formation de gel dans cette phase, la présence des précurseurs de coacervation dans la phase aqueuse ne garantit pas leur placement adéquat à l'interphase entre la phase aqueuse et la phase huileuse, lorsque la polymérisation est démarrée.

Par suite, l'écorce formée n'assure pas toujours une rétention satisfaisante de la phase dispersée et peut parfois présenter une épaisseur importante.

Un but de l'invention est de disposer d'un procédé de formation d'une pluralité de gouttes d'une première phase, dispersées dans une deuxième phase, qui permet d'obtenir des gouttes très stables stabilisées par une écorce très fine.

Un autre but de l'invention est d'obtenir un procédé de fabrication d'une dispersion stable de gouttes de première phase dans une deuxième phase qui puisse être très concentrée, tout en restant apte à s'écouler, notamment à des faibles cisaillements.

A cet effet, l'invention a pour objet un procédé selon la revendication 1.

Le procédé selon l'invention peut comprendre l'une ou plusieurs des caractéristiques des revendications 2 à 13 ou des caractéristiques suivantes, pris(es) isolément ou suivant toute combinaison techniquement possible :

- le deuxième conduit débouche en aval dans un réceptacle en étant immergé dans un liquide reçu dans le réceptacle ;
- l'autre du premier précurseur et du deuxième précurseur est choisi parmi un polymère hydrophile cationique tel que le chitosan ou les dérivés de gomme guar, notamment le chlorure d'hydroxypropyltrimonium guar ;
- l'étape de formation de gouttes comporte l'amenée d'un volume continu de premier fluide et d'un volume continu de deuxième fluide, puis l'agitation du premier fluide et du deuxième fluide pour former une dispersion de la première phase dans la deuxième phase ;
- l'étape d'amenée du deuxième polymère précurseur comporte l'ajout d'un fluide contenant le deuxième polymère précurseur dans le deuxième fluide, et l'agitation de ce fluide contenant le deuxième polymère précurseur pour le disperser dans la deuxième phase contenant les gouttes de première phase ;
- l'écorce présente une épaisseur inférieure à 1 $\mu$m notamment inférieure à 100 nm avantageusement inférieure à 50 nm, en particulier inférieure à 10 nm ;
- l'écorce présente une épaisseur comprise entre 1 nm et 500 nm.

L'invention sera mieux comprise à la lecture de la description qui va suivre, donnée uniquement à titre d'exemple, et faite en se référant aux dessins annexés, sur lesquels :

- la figure 1 est une vue schématique de côté d'une première dispersion obtenue par un procédé selon l'invention, contenue dans un récipient ;
- la figure 2 est une vue agrandie d'une goutte de première phase contenue dans la deuxième phase et présentant une écorce de coacervat, la goutte ayant été obtenue par un premier procédé selon l'invention ;
- la figure 3 est une vue schématique de côté d'un premier appareil de mise en oeuvre du procédé selon l'invention,

lors de la mise en oeuvre de ce procédé ;

- la figure 4 est une vue agrandie de la buse de formation de gouttes du premier appareil de mise en oeuvre du procédé selon l'invention ;
- la figure 5 est une vue analogue à la figure 4 d'une variante de buse ;
- la figure 6 est une vue schématique illustrant les différentes étapes d'un deuxième procédé de formation selon l'invention ;
- la figure 7 est une vue analogue à la figure 3 d'une variante d'appareil de mise en oeuvre ; et
- la figure 8 est une vue agrandie de la buse de formation de gouttes de la figure 7.

Les figures 1 à 4 illustrent la mise en oeuvre d'un premier procédé de formation de gouttes selon l'invention.

En référence à la Figure 1, ce premier procédé est destiné à former une dispersion 10 de gouttes 12 d'une première phase 14 dispersée dans une deuxième phase 16 sensiblement immiscible avec la première phase 14.

Dans cet exemple, la première phase 14 est une phase aqueuse. Elle contient notamment sous forme liquide un premier produit qui est choisi parmi un produit biologiquement actif, un produit cosmétique, ou un produit comestible propre à être consommé.

Lorsque le premier produit est un produit biologiquement actif, il est choisi avantageusement parmi les anticoagulants, les anti-thrombogéniques, les agents antimitotiques, les agents anti-prolifération, antiadhésion, anti-migration, les promoteurs d'adhésion cellulaire, les facteurs de croissance, les molécules antiparasitaires, les anti-inflammatoires, les angiogéniques, les inhibiteurs de l'angiogenèse, les vitamines, les hormones, les protéines, les antifongiques, les molécules antimicrobiennes, les antiseptiques ou les antibiotiques.

En variante, la première phase 14 contient des agents réactifs tels que des protéines ou des réactifs destinés à former un bioréacteur, ou à former des cellules artificielles pour des implants.

Un produit cosmétique pouvant être contenu dans le coeur est par exemple cité dans la Directive 93/35/CEE du Conseil datée du 14 juin 1993. Ce produit est par exemple une crème, une émulsion, une lotion, un gel et une huile pour la peau (mains, visage, pieds, etc.), un fond de teint (liquide, pâte) une préparation pour bains et douches (sels, mousses, huiles, gels, etc.), un produit de soins capillaires (teintures capillaires et décolorants), un produit de nettoyage (lotions, poudres, shampoings), un produit d'entretien pour la chevelure (lotions, crèmes, huiles), un produit de coiffage (lotions, laques, brillantines), un produit pour le rasage (savons, mousses, lotions, etc.), un produit destiné à être appliqué sur les lèvres , un produit solaire, un produit de bronzage sans soleil, un produit permettant de blanchir la peau, un produit antirides.

Les produits comestibles propres à être consommés par un être humain ou par un animal sont avantageusement des purées de légumes ou de fruits telles que la purée de mangue, de la purée de poire, de la purée de coco, de la crème d'oignons, de poireaux, de carottes, ou d'autres préparations pouvant mélanger plusieurs fruits ou légumes. En variante, il s'agit d'huiles telles qu'une huile alimentaire, du type huile d'olive, huile de soja, huile de grains de raisin, huile de tournesol, ou toute autre huile extraite des végétaux.

La deuxième phase 16 est une huile. Cette phase est réalisée par exemple à partir d'une huile liquide, de viscosité inférieure à 20000mPa.s. de préférence en dessous de 1000mPa.s.

L'huile constituant la phase huileuse est par exemple une huile siliconée, une huile minérale, une huile végétale ou un mélange de ces huiles.

La deuxième phase 16 huileuse comprend, dans une variante, des molécules d'intérêt cosmétique, tels que des actifs, des colorants, des stabilisants, des conservateurs, des agents modificateurs choisis parmi des agents de texture, de viscosité, de pH, de force osmotique ou des modificateurs d'indice de réfraction.

Avantageusement, la dispersion 10 est sensiblement translucide.

Par sensiblement translucide, on entend que l'absorbance de la dispersion selon l'invention est généralement inférieure à 5%, de préférence inférieure 2%, préférentiellement inférieure à 1% pour au moins une longueur d'onde dans le spectre visible compris de 400 nanomètres à 1000 nanomètres, avantageusement sur toute longueur d'onde du spectre visible de 400 nanomètres à 1000 nanomètres.

L'intensité transmise à travers la dispersion 10 selon l'invention est divisée par un facteur d'au moins $10^3$ en comparaison avec une émission concentrée classique.

Cette translucidité est mesurée en introduisant un échantillon de dispersion dans une cuve de 2 mm de trajet optique à une longueur d'onde comprise entre 400 nanomètres et 1000 nanomètres.

La première phase 14 et la deuxième phase 16 sont sensiblement immiscibles. Ainsi, la solubilité de la première phase 14 dans la deuxième phase 16 est inférieure à 5 % en masse.

Le procédé selon l'invention permet d'obtenir une pluralité de gouttes 12 de première phase 14 tel qu'illustré sur la figure 2. Chaque goutte 12 comporte ainsi un coeur 17 constitué de première phase 14 et une écorce 18 de retenue et de stabilisation du coeur 17, l'écorce 18 étant formée par un coacervat entre un premier polymère précurseur et un deuxième polymère précurseur, comme décrit en détail plus bas.

Dans l'exemple représenté sur la figure 1 chaque goutte 12 présente une densité supérieure à celle de la

deuxième phase 16 dans laquelle elle est dispersée.

Ainsi, les gouttes 12 s'accumulent au fond du récipient 20 qui reçoit la dispersion 10. Les gouttes 12 sont disposées en appui les unes sur les autres. Par suite, la dispersion comprend au moins une région concentrée 22 comportant des gouttes 12 et au moins une région 24 dépourvue de gouttes 12 et comprenant exclusivement de la deuxième phase 16.

Dans l'exemple représenté sur la figure 1, la concentration de gouttes dans la région concentrée 22 est élevée, de sorte que la teneur volumique en première phase dans la région concentrée 22 est supérieure à 50 % et est notamment supérieure à 60 %.

Dans cet exemple, le diamètre des gouttes 12 est supérieur à 500 μm, et est avantageusement inférieur à 3000 μm.

De manière avantageuse, le diamètre des gouttes est compris entre 500 microns et 2500 microns. Les gouttes 12 sont visibles dans la phase aqueuse 16.

Dans un mode de réalisation, lorsque les gouttes de la dispersion possèdent une taille supérieure à 500 μm, l'invention propose de fournir une dispersion de type eau dans huile dont les gouttes 12 présentent une distribution de taille uniforme.

Plus précisément, selon ce mode, la phase aqueuse dispersée est constituée d'une population de gouttes monodisperses telles qu'elles possèdent un diamètre moyen $\overline{D}$ compris de 500 μm à 3 000 μm et un coefficient de variation $C_v$ inférieur à 10%.

Dans le cadre de la présente description, on entend par « gouttes monodispersées » le fait que la population de gouttes dispersées de la dispersion selon l'invention possède une distribution de taille uniforme. Des gouttes monodispersées présentent une bonne monodispersité. A l'inverse, des gouttes présentant une mauvaise monodispersité sont dites « polydispersées ».

Le diamètre moyen $\overline{D}$ des gouttes est par exemple mesuré par analyse d'une photographie d'un lot constitué de N gouttes, par un logiciel de traitement d'image (Image J). Typiquement, selon cette méthode, le diamètre est mesuré en pixel, puis rapporté en μm, en fonction de la dimension du récipient contenant les gouttes la dispersion.

De préférence, la valeur de N est choisie supérieure ou égale à 30, de sorte que cette analyse reflète de manière statistiquement significative la distribution de diamètres des gouttes de ladite émulsion.

On mesure le diamètre $D_i$ de chaque goutte, puis on obtient le diamètre moyen $\overline{D}$ en calculant la moyenne arithmétique de ces valeurs $D_i$ :

$$\overline{D} = \frac{1}{N}\sum_{i=1}^{N} D_i$$

A partir de ces valeurs $D_i$, on peut également obtenir l'écart-type $\sigma$ des diamètres des gouttes de la dispersion :

$$\sigma = \sqrt{\frac{\sum_{i=1}^{N}\left(D_i - \overline{D}\right)^2}{N}}$$

L'écart-type $\sigma$ d'une dispersion reflète la répartition des diamètres $D_i$ des gouttes de la dispersion autour du diamètre moyen $\overline{D}$.

En connaissant le diamètre moyen $\overline{D}$ et l'écart-type $\sigma$ d'une dispersion, on peut déterminer que l'on trouve 95% de la population de gouttes dans l'intervalle de diamètres [$\overline{D}$ - 2$\sigma$, $\overline{D}$ + 2$\sigma$] et que l'on trouve 68% de la population dans l'intervalle [$\overline{D}$ - $\sigma$, $\overline{D}$ + $\sigma$].

Pour caractériser la monodispersité de la dispersion selon ce mode de l'invention, on peut calculer le coefficient de variation $C_v$ :

$$C_v = \frac{\sigma}{\overline{D}}$$

Ce paramètre reflète la répartition des diamètres des gouttes en fonction du diamètre moyen de celles-ci.

Le coefficient de variation des diamètres des gouttes de la dispersion selon ce mode de l'invention est inférieur à 10%, de préférence inférieur à 5%.

Alternativement, la monodispersité peut être mise en évidence en plaçant un échantillon de dispersion 10 dans un flacon à section circulaire constante. Une agitation douce par rotation de un quart de tour sur une demi-seconde autour de l'axe de symétrie traversant le flacon, suivie d'un repos d'une demi-seconde est effectuée, avant de répéter l'opération en sens inverse, et ce quatre fois de suite.

Les gouttes 12 de la dispersion s'organisent sous une forme cristalline lorsqu'elles sont monodisperses. Ainsi, elles présentent un empilement suivant un motif se répétant suivant dans les trois dimensions. Il est alors possible d'observer, un empilement régulier qui indique une bonne monodispersité, un empilement irrégulier traduisant la polydispersité de la dispersion.

Lorsque la dispersion est laissée au repos, les gouttes 12 sont stables et n'adhèrent pas les unes aux autres, aucune coalescence n'est observée après 2 semaines à 40°C.

La dispersion obtenue par un procédé selon l'invention supporte une agitation sans cisaillement sans subir une coalescence importante des gouttes 12 ni une altération de sa monodispersité.

Pour tester cette propriété, un échantillon de la dispersion selon l'invention est placé dans un récipient de 2 mL, puis celui-ci est placé dans une alvéole d'un plateau d'agitation (IKA vortex Genius 3). La taille d'une alvéole est environ 50% plus grande que celle du récipient contenant l'échantillon. Ainsi, lors de l'agitation le récipient heurte les parois de l'alvéole, ce qui créé un grand nombre de chocs. La vitesse d'agitation est d'environ 500 rpm. Le test est considéré comme réussi lorsque moins de 5% de gouttes en nombre ont subi une coalescence à l'issue d'une heure d'agitation sans cisaillement.

En plus des propriétés énoncées plus haut, de telles gouttes sont non élastiques, et présentent un comportement granulaire lorsque la dispersion est agitée, de sorte qu'elles s'écoulent les unes sur les autres tels des objets solides et qu'elles adoptent un comportement particulaire en suspension.

En outre, la dispersion obtenue par un procédé selon l'invention peut présenter les propriétés suivantes.

Selon certains modes de réalisation avantageux, la dispersion obtenue par un procédé selon l'invention supporte une chute d'une hauteur d'un mètre sans subir une coalescence importante des gouttes, ni une altération de sa monodispersité.

Pour tester cette propriété, un échantillon de la dispersion obtenue par un procédé selon l'invention est placé dans un récipient de 2 mL, puis celui-ci est lâché en haut d'un tube en verre d'un mètre de hauteur servant de guide, sur un substrat solide, et récupéré à son extrémité inférieure. L'opération est répétée trois fois. Le test est considéré comme réussi lorsque moins de 5% de gouttes 12 en nombre ont subi une coalescence à l'issue des trois chutes.

Les gouttes 12 sont sensiblement non élastiques. La non-élasticité des gouttes peut être caractérisée par un seuil de résistance à l'écoulement très faible, mesurée par exemple par la méthode suivante : un échantillon de dispersion 10 est placé dans un rhéomètre de type rhéometrics RFSII utilisant une géométrie cône-plan ayant un entrefer de 45 micromètres. Les cisaillements étudiés sont faibles, et sont notamment compris entre $1s^{-1}$ et $10s^{-1}$. Le module de cisaillement est mesuré pour des cisaillements croissants entre $1s^{-1}$ et $10s^{-1}$.

Ainsi, le module de cisaillement, sous un cisaillement faible, notamment égal à 2 $s^{-1}$, est inférieur à 200 Pa.s, et est notamment inférieur à 100 Pa.s.

Ce module de cisaillement est inférieur d'au moins $10^4$ au module de cisaillement observé au même cisaillement, pour une dispersion classique stabilisée par des tensioactifs et présentant la même composition en huile et en eau.

En comparaison avec une émulsion classique (huile/eau) monodisperse concentrée à 80% (cf. Mason et al. J. Coll. Int. Sci. 179, 439-448 (1996)), une émulsion concentrée selon ce mode de réalisation présente une viscosité $\eta$ (Pa.s) inférieure d'un facteur $10^4$, pour un cisaillement allant de 1 à 12 $s^{-1}$.

Le coeur 17, même entouré de l'écorce 18, est sensiblement liquide ou peu gélifié. En variante, le coeur est gélifié.

Grâce au procédé selon l'invention, les gouttes 12 obtenues par ce procédé présentent une écorce 18 très fine, notamment d'épaisseur inférieure à 1 % du diamètre des gouttes 12.

L'épaisseur de l'écorce 18 est ainsi inférieure à 1 $\mu$m et est trop faible pour être mesurée par des méthodes optiques. Cette taille est généralement comprise entre 1 nm et 500 nm, de préférence inférieure à 100 nm, avantageusement inférieure à 50 nm, préférentiellement inférieure à 10 nm.

La mesure de l'épaisseur de l'écorce des gouttes de l'invention peut être effectuée par la méthode de diffusion de neutrons aux petits angles (Small-Angle X-ray Scattering), telle que mise en oeuvre dans Sato et al. J. Chem. Phys. 111, 1393-1401 (2007).

Pour cela, les gouttes sont produites en en utilisant de l'eau deutérée, puis sont lavées trois fois avec une huile deutérée, comme par exemple une huile deutérée de type hydrocarboné (octane, dodécane, hexadécane).

Après lavage, les gouttes sont ensuite transférées dans la cellule de Neutrons afin de déterminer le spectre I(q) ; q étant le vecteur d'onde.

A partir de ce spectre, on applique les traitements analytiques classiques (REF) afin de déterminer l'épaisseur de l'écorce hydrogénée (non deutérée).

L'écorce 18 entourant les gouttes de l'émulsion selon l'invention est rigidifiée, ce qui présente pour avantage

de conférer une résistance supérieure aux gouttes et de diminuer, voire d'empêcher, leur coalescence.

Cette écorce est typiquement formée par coacervation, c'est-à-dire par précipitation de polymères chargés de charges opposées. Au sein d'un coacervat, les liaisons liant les polymères chargés entre eux sont de type ionique, et sont généralement plus fortes que des liaisons de type électrostatique présentes au sein d'une membrane de type tensioactif.

L'écorce 18 est formée par coacervation d'au moins deux polymères chargés de polarité opposée (ou polyélectrolyte) et de préférence en présence d'un premier polymère, de type anionique, et d'un deuxième polymère, différent du premier polymère, de type cationique.

Dans le cadre de la présente description, on entend par « polymère de type anionique » un polymère comportant des fonctions chimiques de type anionique. On peut aussi parler de polyélectrolyte anionique.

Par « fonction chimique de type anionique », on entend une fonction chimique AH capable de céder un proton pour donner une fonction A. Selon les conditions du milieu dans lequel il se trouve, le polymère de type anionique comporte donc des fonctions chimiques sous forme AH, ou bien sous forme de sa base conjuguée $A^-$.

Comme exemple de fonctions chimiques de type anionique, on peut citer les fonctions acide carboxylique -COOH, éventuellement présentes sous forme d'anion carboxylate $-COO^-$.

Comme exemple de polymère de type anionique, on peut citer tout polymère formé par la polymérisation de monomères dont au moins une partie porte des fonctions chimiques de type anionique, tel que des fonctions acide carboxylique. De tels monomères sont par exemple l'acide acrylique, l'acide maléique, ou tout monomère éthyléniquement insaturé comportant au moins une fonction acide carboxylique.

Parmi les exemples de polymère de type anionique appropriés à la mise en oeuvre de l'invention, on peut citer les copolymères d'acide acrylique ou d'acide maléique et d'autres monomères, tels que l'acrylamide, les acrylates d'alkyle, les acrylates d'alkyle en $C_5$-$C_8$, les acrylates d'alkyle en $C_{10}$-$C_{30}$, les méthacrylates d'alkyle en $C_{12}$-$C_{22}$, les méthacrylates méthoxypolyéthylèneglycol, les acrylates d'hydroxyester.

Dans le cadre de la présente description, on entend par « polymère de type cationique » un polymère comportant des fonctions chimiques de type cationique. On peut aussi parler de polyélectrolyte cationique.

Par « fonction chimique de type cationique », on entend une fonction chimique B capable de capter un proton pour donner une fonction $BH^+$. Selon les conditions du milieu dans lequel il se trouve, le polymère de type cationique comporte donc des fonctions chimiques sous forme B, ou bien sous forme $BH^+$, son acide conjugué.

Comme exemple de fonctions chimiques de type cationique, on peut citer les fonctions amine primaire, secondaire et tertiaire, éventuellement présentes sous forme de cations ammoniums.

Comme exemple de polymère de type cationique, on peut citer tout polymère formé par la polymérisation de monomères dont au moins une partie porte des fonctions chimiques de type cationique, tel que des fonctions amine primaire, secondaire ou tertiaire.

De tels monomères sont par exemple l'aziridine, ou tout monomère éthyléniquement insaturé comportant au moins une fonction amine primaire, secondaire ou tertiaire.

Parmi les exemples de polymère de type cationique appropriés à la mise en oeuvre de l'invention, on peut citer l'amodiméthicone, dérivé d'un polymère silicone (polydiméthylsiloxane, aussi appelé diméthicone), modifié par des fonctions amine primaire et amine secondaire :

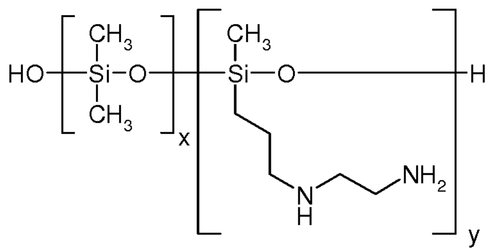

Amodiméthicone

On peut également citer des dérivés de l'amodiméthicone, comme par exemple des copolymères de l'amodiméthicone, l'aminopropyl diméthicone, et plus généralement des polymères silicones comportant des fonctions amines.

On peut citer le copolymère de bis-isobutyl PEG-14/amodiméthicone et le bis-hydroxy/méthoxy amodiméthicone.

On peut également citer les polymères de type polysaccharide comprenant des fonctions amine, tel que le chitosan ou les dérivés de gomme guar (chlorure d'hydroxypropyltrimonium guar).

On peut également citer les polymères de type polypeptide comprenant des fonctions amine, tel que la polylysine.

On peut également citer les polymères de type polyéthylèneimine comprenant des fonctions amine, tel que la polyéthylèneimine linéaire ou branchée.

La coacervation a généralement lieu en présence d'un premier polymère de type anionique et d'un deuxième polymère de type cationique, qui jouent le rôle d'agents de rigidification de la membrane.

La formation du coacervat entre ces deux polymères est généralement provoquée par une modification des conditions du milieu réactionnel (température, pH, concentration en réactifs, etc.). La réaction de coacervation résulte de la neutralisation de ces deux polymères chargés de polarités opposées et permet la formation d'une structure membranaire par interactions électrostatiques entre le premier et le deuxième polymère. La membrane ainsi formée autour de chaque goutte encapsule totalement le coeur 17 et isole la première phase 14 de la deuxième phase 16.

Comme on le verra plus bas dans la description du procédé selon l'invention, le premier polymère est contenu initialement dans l'une de la première phase 14 et de la deuxième phase 16, le deuxième polymère étant contenu initialement, avant la formation des gouttes 12, dans l'autre de la première phase 14 et de la deuxième phase 16. Les deux polymères migrent ensuite à l'interface lors de la formation des gouttes où ils forment l'écorce 18 par coacervation.

Une dispersion avantageuse est telle que chaque goutte comprend, en masse par rapport à la masse de ladite goutte :

- de 0,05% à 10% d'un polymère $P_1$ de type anionique et hydrophile, et
- de 0,05% à 10% d'un polymère $P_2$ de type cationique et lipophile.

De préférence, chaque goutte comprend, en masse par rapport à la masse de ladite goutte, de 0,1% à 5% d'un polymère $P_1$ de type anionique et hydrophile.

De préférence, chaque goutte comprend, en masse par rapport à la masse de ladite goutte, de 0,1% à 5% d'un polymère $P_2$ de type cationique et lipophile.

Une dispersion avantageuse selon cette variante est telle que chaque goutte comprend un polymère $P_1$, de type anionique et hydrophile, et un polymère $P_2$, de type cationique et lipophile, dans un rapport massique $P_1$:$P_2$ compris entre 1:10 et 10:1.

Le premier procédé selon l'invention est mis en oeuvre à l'aide d'une méthode microfluidique dans un appareil 30 illustré par les figures 3 et 4.

Cet appareil 30 comporte une buse 32 de formation des gouttes et un réceptacle 33 de réception des gouttes formées.

La buse de formation 32 comporte un conduit interne 34 d'amenée d'un fluide interne 36 comprenant la première phase 14, et un conduit externe 38 disposé autour du conduit interne 36 pour amener un fluide externe 40 formant au moins une partie de la deuxième phase 16.

Dans l'exemple illustré par les figures 4 et 5, la buse 32 comprend en outre avantageusement un conduit 42 d'amenée d'un fluide réactionnel 44 débouchant dans le conduit externe 36.

L'appareil 30 comporte de plus des moyens 46 d'amenée de fluide interne 36 dans le conduit interne 34, des moyens 48 d'amenée de fluide externe 40 dans l'espace annulaire délimité entre le conduit interne 34 et le conduit externe 38 et des moyens 50 d'amenée de fluide réactionnel 44 dans le conduit d'amenée 42 de fluide réactionnel lorsque celui-ci est présent.

Dans l'exemple représenté sur les figures 3 et 4, le diamètre maximal des conduits 34, 38 et 42 est inférieur à 3 mm pour préserver le caractère microfluidique du procédé.

Le conduit interne 34 est avantageusement disposé coaxialement dans le conduit externe 38. Il est raccordé en amont aux moyens d'amenée 46. Il débouche en aval par une ouverture aval 52 disposée dans le conduit externe 38, en retrait par rapport à l'ouverture aval 54 définie par le conduit externe 38, au dessus de cette ouverture 54.

Ainsi, la distance séparant l'ouverture aval 52 du conduit interne 34 et l'ouverture aval 54 du conduit externe 38 est de préférence supérieure à 1 fois le diamètre du conduit externe 38.

Le conduit externe 38 délimite avec le conduit interne 34 un espace annulaire raccordé en amont aux moyens d'amenée 48.

L'ouverture aval 54 du conduit externe 38 est située au-dessus et à l'écart du récipient 33. Elle débouche ainsi dans un volume d'air.

Le conduit d'amenée 42 débouche transversalement dans le conduit externe 38, en aval de l'ouverture aval 52 du conduit interne 36 et en amont de l'ouverture aval 54 du conduit externe 38. Il est raccordé en amont aux moyens d'amenée 50.

Les moyens d'amenée 46, 48 et 50 comportent chacun par exemple un pousse seringue, une pompe péristaltique ou un autre système générateur de pression contrôlant le débit, comme par exemple un pot à pression couplé d'un débitmètre et d'un système de régulation du débit.

Chacun des moyens d'amenée 46, 48, 50 est propre à convoyer un fluide respectif 36, 40, 44 à un débit contrôlé et réglable.

Le récipient 33 est disposé au-dessous de l'ouverture aval 54. Il contient un volume 55 de liquide destinée à former une autre partie de la deuxième phase 16, avantageusement un volume de fluide externe 40.

La surface supérieure du volume 55 de fluide est située axialement à l'écart de l'ouverture aval 54, prise le long de l'axe A-A', de sorte que les gouttes 12 formées dans la buse 32 chutent sous l'effet de leur poids à travers un volume d'air entre l'ouverture aval 54 et la surface supérieure 56 du volume 55 de liquide.

Dan une variante (non représentée), l'ouverture aval 54 est plongée dans le volume de fluide 55.

Dans l'exemple représenté sur la figure 3, le dispositif 30 a été illustré avec une seule buse 32.

Dans une variante avantageuse, le système 30 comporte une pluralité de buses 32 disposées en parallèle au dessus et en regard d'un récipient 33.

Un premier procédé selon l'invention va maintenant être décrit.

Initialement, le fluide interne 36 est préparé en mélangeant la première phase 14 destinée à former le coeur 17 de la goutte 12 et le premier polymère précurseur de la coacervation.

Dans cet exemple, le fluide interne 36 est avantageusement aqueux et le premier polymère est un polymère hydrosoluble, par exemple de type anionique.

Parallèlement, le fluide externe 40 est également préparé en mélangeant un solvant huileux et éventuellement un agent stabilisateur formé par exemple par une résine hydrophobe.

L'agent stabilisateur se place typiquement à l'interface entre la phase huileuse et la phase aqueuse, stabilisant les gouttes et évitant leur coalescence avant la formation de l'écorce.

L'agent stabilisateur est par exemple une résine hydrophobe. Cette résine peut être par exemple un silicate, telle qu'une résine de triméthylsiloxysilicate.

La teneur massique en agent stabilisateur est par exemple comprise entre 10% et 0,001 %.

Dans cet exemple de procédé figures 4, 5, 6, 7 et 8, un fluide réactionnel 44 est également préparé. Ce fluide contient un deuxième polymère précurseur de la coacervation, avantageusement dissout dans la même huile que celle présente dans le fluide externe 40.

Le fluide réactionnel 44 comprend en outre un agent réactionnel propre à engendrer la coacervation du premier polymère avec le deuxième polymère, tel qu'un agent susceptible de modifier le pH comme un acide ou une base.

Dans cet exemple, l'agent réactionnel est une base.

Puis, le fluide interne 36, le fluide externe 40, et le fluide réactionnel 44 sont disposés respectivement dans les moyens d'amenée respectifs 46, 48 et 50.

De même, une masse liquide 55 formée d'un solvant huileux de nature analogue à celle du fluide externe 40 est introduit dans le récipient 33.

Ensuite, les moyens d'amenée 46, 48 et 50 sont activés.

Le flux de fluide interne 36 circulant dans le conduit interne 34 engendre des gouttes 12 de première phase 14 au niveau de l'ouverture aval 52 du conduit interne lorsque le fluide interne 36 passe dans le fluide externe 40.

Les gouttes 12 circulent alors dans le fluide externe 40 vers l'ouverture 54. Elles entrent en contact avec le fluide réactionnel 44 introduit à travers le conduit d'amenée 42.

Le premier polymère présent dans le fluide interne 36 migre à l'interface entre le fluide interne 36 et le fluide externe 40. De même, le deuxième polymère présent dans le fluide externe 40 migre à l'interface entre le fluide externe 40 et le fluide interne 36.

Sous l'action de l'agent réactionnel, la coacervation entre le premier polymère et le deuxième polymère se produit pour former l'écorce 18.

Le premier polymère et le deuxième polymère n'étant pas présents initialement dans la même phase, le risque qu'ils réagissent prématurément, notamment avant la formation des gouttes 12 de fluide interne 36 dans le fluide externe 40, garantit que l'écorce 18 formée à l'interface entre la phase interne 14 et la phase externe 16 est complète, très mince, et n'engendre pas de gélification totale du coeur 17.

Les gouttes 12 ainsi formées sont donc très stables, peu ou pas élastiques et n'ont pas tendance à coalescer les unes sur les autres.

Au moins une goutte 12 formée dans le conduit externe 38 est ensuite reçue dans une goutte extérieure 60 de fluide externe 40 qui est formée à la sortie du conduit externe 38, au niveau de l'ouverture aval 54. La goutte extérieure 60 tombe dans le volume 55 à travers un volume d'air et les gouttes 12 de phase interne 14 sédimentent dans la deuxième phase 16 formée par le fluide externe 40 des gouttes 60 et par le volume 55.

Le procédé selon l'invention est donc particulièrement efficace pour former des gouttes stables, de dimensions supérieures à 500 $\mu$m, sans l'utilisation de tensio-actifs et de manière particulièrement contrôlée.

La figure 5 illustre une buse 32 pour une variante d'appareil 30 de formation de gouttes 12 selon l'invention.

A la différence de la buse 32 représentée sur la figure 4, le conduit externe 38 comprend un tronçon amont 70 et un tronçon aval 72, le tronçon amont 70 faisant saillie partiellement dans le tronçon aval 72.

Le tronçon amont 70 présente une section transversale inférieure à la section transversale du tronçon aval 72. Le conduit d'amenée 42 débouche en aval dans l'espace annulaire délimité entre le tronçon amont 70 et le tronçon aval 72.

Par ailleurs, le contour de la section transversale du tronçon amont 70 n'est pas nécessairement homothétique au contour de la section transversale du tronçon aval. Ainsi, le tronçon amont 70 peut présenter une section transversale circulaire et le tronçon aval une section transversale polygonale, par exemple carrée ou rectangle.

La mise en oeuvre du procédé selon l'invention dans cette variante d'appareil 30 est par ailleurs analogue à celle du premier procédé.

Un deuxième procédé selon l'invention est décrit en regard de la figure 6. Ce procédé est mis en oeuvre en « batch » dans un récipient 20.

Initialement, à l'étape (a), un premier volume continu de fluide interne 36 est disposé dans le récipient 20. Comme décrit précédemment, le fluide interne 36 contient la première phase 14 et le premier polymère. Puis, à l'étape (b), un deuxième volume continu de fluide externe 40 est introduit dans le récipient 20 au-dessus du fluide interne 36. Les deux volumes sont alors sensiblement séparés sans être significativement dispersés l'un dans l'autre.

Puis, le récipient 20 est agité pour produire une dispersion de gouttes 12 de fluide interne 36 dans du fluide externe 40 (étape (c) sur la figure 6). Lors de cette étape, le premier polymère précurseur présent dans le fluide interne 36 migre à l'interface entre les gouttes 12 de fluide interne 36 et le fluide externe 40. Puis, une quantité déterminée de fluide réactionnel est ajoutée dans le récipient 20 (étape (d) sur la figure 6). Ce fluide réactionnel contient l'agent réactionnel et le deuxième polymère précurseur.

De même, le deuxième polymère présent dans la phase externe 40 migre à l'interface entre les gouttes 12 de fluide interne 36 et la phase externe 40.

L'agent réactionnel migre alors à l'interface entre les gouttes de fluide interne 36 et le fluide externe 40 et déclenche la coacervation entre le premier polymère et le deuxième polymère pour former l'écorce 18.

Afin d'améliorer cette dispersion, le récipient 20 est à nouveau agité (étape (e)).

Comme décrit précédemment, le premier polymère précurseur et le deuxième polymère n'étant mis en contact qu'après formation des gouttes 12, ou lors de cette formation, le procédé évite la formation importante de gel dans les gouttes 12 et permet la formation d'une écorce 18 de taille réduite.

Comme décrit plus haut, le premier polymère est disposé dans un premier fluide contenant la première phase 14 et le deuxième polymère est inclus dans un fluide immiscible avec la première phase. Le deuxième polymère peut être ajouté dans le deuxième fluide dans lequel les gouttes 12 sont formées ou dans un fluide réactionnel ajouté éventuellement après formation des gouttes 12.

Des exemples de mise en oeuvre du procédé vont maintenant être décrits.

Exemple 1

Sur le dispositif expérimental de la figure 3, on met en oeuvre une étape de rigidification en ligne entre un premier polymère, de type anionique et hydrophile (acide polyacrylique), et un deuxième polymère, de type cationique et lipophile (amodiméthicone).

Le premier polymère est contenu dans le fluide interne 36 (pH compris entre 3 et 4) et le deuxième polymère est contenu dans fluide réactionnel (qui contient également une base), qui est apporté par voie perpendiculaire aux deux autres, via un connecteur en T positionné à l'extrémité du tube de collecte.

L'étape de rigidification est basée sur la formation d'un coacervat au niveau de l'interface entre l'acide polyacrylique contenu dans le fluide interne et un amino-silicone (Amodimethicone) apportée par le fluide réactionnel, après formation des gouttes.

La rencontre de ces deux polymères provoque la coacervation et la rigidification de la membrane autour des gouttes.

Sur ce dispositif expérimental décrit on applique les débits suivant : FE = 6 000 [$\mu$L/heure, FI = 4 000 $\mu$L/heure et FR = 2 000 $\mu$L/heure La composition de chaque fluide est décrite dans le Tableau ci-dessous.

| Phase | Nom du produit | % massique | Fonction |
|---|---|---|---|
| Fluide externe | PDMS 6 sCt | 98 % | Solvant |
| | DC 749 | 2 % | Agent stabilisateur |
| Fluide interne | Eau | 99,5% | Solvant |
| | Acide polyacrylique | 0,5% | Agent de rigidification |

(suite)

| Phase | Nom du produit | % massique | Fonction |
|---|---|---|---|
| Fluide réactionnel | PDMS 6 sCt | 96 % | solvant |
| | Amino-silicone | 2% | Agent de rigidification |
| | Dibutyléthanolamine | 2% | Base |
| PDMS 6 cSt = polydiméthylsiloxane (diméthicone) de viscosité 6 cSt<br>DC 749 = mélange 50/50 de cyclométhicone (cyclopentasiloxane) et de triméthylsiloxysilicate | | | |

On obtient à la suite de la collecte un flacon contenant des gouttes d'environ 900 μm de diamètre ayant une taille uniforme suivant les méthodes décrites plus haut.

Les gouttes sont stables au test d'agitation sans cisaillement et au test d'agitation sous cisaillement (moins de 5% de gouttes subissent une coalescence). De même aucune coalescence n'est observée après 2 semaines à 40°C.

L'échantillon supporte une chute d'un mètre sur un substrat solide.

Les gouttes sont non adhésives et glissent les unes sur les autres lorsque l'émulsion est agitée.

Exemple 2

Cet exemple met en avant une étape de rigidification en ligne entre un premier polymère, de type anionique et hydrophile (acide polyacrylique), et un deuxième polymère, de type cationique et lipophile (amodiméthicone).

La fabrication se fait dans le même dispositif que celui de l'exemple 1.

L'étape de rigidification est basée sur la formation d'un coacervat au niveau de l'interface entre l'acide polyacrylique contenu dans le fluide interne et un amino-silicone apporté par le fluide réactionnel, après formation des gouttes. Sur le dispositif expérimental décrit, on applique les débits suivants : FE=13000 μl/h, FI=5000 μl/heure et FR=1250 μl/heure. La composition de chaque fluide est décrite dans le tableau ci-dessous.

| Phase | Nom du produit | % massique | Fonction |
|---|---|---|---|
| Fluide externe | PDMS 6 sCt | 98,5% | Solvant |
| | PEG10-diméthicone | 0,1% | Agent stabilisateur |
| | Diéthyléthanolamine | 0,05% | Base |
| Fluide interne | Eau | 99,5% | Solvant |
| | Acide polyacrylique | 0,5% | Agent de rigidification |
| Fluide réactionnel | PDMS 6 sCt | 99% | solvant |
| | Amino-silicone | 1% | Agent de rigidification |

Exemple 3 Dispositif de rigidification en batch

Cet exemple met en oeuvre une étape de rigidification en batch entre un premier polymère, de type anionique et hydrophile de type carbomer (acide polyacrylique), et un deuxième polymère, de type cationique et lipophile (amodiméthicone).

Le premier polymère est contenu dans le fluide interne (pH compris entre 3 et 4) et le deuxième polymère est contenu dans fluide réactionnel (qui contient également une base). La composition de chaque fluide est décrite dans le Tableau ci-dessous :

| Phase | Nom du produit | % massique | Fonction |
|---|---|---|---|
| Fluide Externe | PDMS 6 sCt | 100% | Solvant |
| Fluide Interne | Eau | 99,75% | Solvant |
| | Carbomer = Acide polyacrylique | 0,25% | Agent de rigidification |

(suite)

| Phase | Nom du produit | % massique | Fonction |
|---|---|---|---|
| Fluide Réactionnel | PDMS 6 sCt | 98,8% | solvant |
| | Amino-silicone | 1% | Agent de rigidification |
| | Diéthyléthanolamine | 0,2% | Base |

Dans un récipient en verre de 4 mL, on ajoute 0,8 mL de fluide interne puis 2 mL de fluide externe. Le récipient est ensuite agité pendant 15 s à 1400 tours par minutes sur un vortex (IKA MS2 minishaker), ce par quoi on forme les gouttes de fluide interne dans le fluide externe. Pour effectuer la rigidification des gouttes formées, on ajoute ensuite 0,2 mL de fluide réacionnel dans le récipient, puis celui-ci est de nouveau agité pendant 15 s à 1400 tours par minutes sur un vortex (IKA MS2 minishaker). La dispersion est ensuite laissée au repos pendant 5 minutes avant d'être utilisée.

A l'issue de ce protocole, on obtient un récipient contenant des gouttes de tailles variées, essentiellement comprises entre 500 $\mu$m et 1000 $\mu$m. Les gouttes sont stables au test d'agitation sans cisaillement et au test d'agitation sous cisaillement (moins de 5% de gouttes subissent une coalescence). De même aucune coalescence n'est observée après 2 semaines à 40°C.

L'échantillon supporte également une chute d'un mètre sur un substrat solide.

Les gouttes sont non adhésives et glissent les unes sur les autres lorsque l'émulsion est agitée.

Dans une variante, la première phase 14 formant les gouttes 12 est une phase huileuse, et la deuxième phase 16 dans laquelle les gouttes 12 sont dispersées est une phase aqueuse.

La phase huileuse est immiscible avec la phase aqueuse. Comme décrit précédemment, les gouttes 12 comportent un coeur 17 constitué de première phase 14 et une écorce de retenue et de stabilisation du coeur 17, l'écorce 18 étant formée par un coacervat entre un premier polymère précurseur et un deuxième polymère précurseur comme décrit précédemment.

Les gouttes 12 présentent un diamètre supérieur à 500 micromètres et avantageusement inférieur à 3000 micromètres, notamment compris entre 800 micromètres et 2000 microns. L'écorce présente une épaisseur inférieure à 1000 nanomètres, notamment comprise entre 1 nanomètres et 500 nanomètres.

Les polymères précurseurs du coacervat sont ceux qui ont été décrits précédemment. Ils ne seront pas décrits plus en détail.

Les gouttes 12 ainsi formées sont avantageusement monodispersées. Elles présentent un coefficient de variation, tel que défini plus haut, inférieur à 10 %.

Selon l'invention, les gouttes présentent une capacité à la déformation supérieure à 50 %, avantageusement à 100 %. Cette capacité à la déformation est caractérisée par le coefficient gamma calculé par l'équation : $\gamma = \frac{A_{MAX} - A_{INIT}}{A_{MAX}}$, dans lequel $A_{MAX}$ constitue l'aire apparente de la goutte 12 juste avant sa rupture, et $A_{INIT}$ l'aire apparente de la goutte 12 initialement mesurée.

Pour caractériser la déformabilité des gouttes 12, le protocole suivant est mis en oeuvre. Une table optique de type « THORLABS » est utilisée comme support du dispositif expérimental de mesure pour isoler au maximum des vibrations ce dispositif expérimental. Une plaque de verre est maintenue à l'horizontale par des pinces à une dizaine de centimètres au dessus de la table optique. Un niveau à bulle circulaire est utilisé pour contrôler l'horizontalité. Un élévateur est disposé sous la plaque en verre en étant immobile grâce à des pinces « THORLABS ». Une feuille de papier blanc est assemblée sur l'élévateur ou un ruban adhésif blanc sur la plaque de verre afin de disposer d'un meilleur contraste. Une lame de microscope est disposée sur l'élévateur et est déplaçable vers la plaque de verre.

Une caméra Véo Discovery VMS 001 reliée à un ordinateur filme le dispositif expérimental par le dessus. Le logiciel d'acquisition utilisé est Astra Image Webcam Vidéo Graveur. Les vidéos enregistrées sont ensuite exploitées avec le logiciel Image J. Les distances en pixels sont converties en millimètres grâce à un étalon de taille connue (par exemple une bille de verre dont la taille précise est mesurée au microscope avec un micromètre).

Initialement, la plaque de verre et la lame sont écartées de quelques centimètres.

Une unique goutte 12 isolée de la dispersion 10 selon l'invention, entourée d'un peu de deuxième phase 16, est posée sur la lame. La lame est ensuite rapprochée de la plaque. Lorsque la deuxième phase 16 commence à mouiller la plaque, la lame est descendue légèrement de manière à obtenir une goutte 12 à la moindre contrainte possible. Une mesure est prise pour déterminer l'aire apparente initiale $A_{INIT}$ de la goutte 12.

Puis, la goutte 12 est comprimée jusqu'à son éclatement, par exemple à une vitesse de rapprochement de la lame vers la plaque sensiblement égale à 0,5 mm/min Juste avant l'éclatement de la goutte 12, par exemple 50 microsecondes avant l'éclatement de la goutte 12, l'aire apparente maximale $A_{MAX}$ de la goutte 12 est mesurée.

L'expérience est ensuite reproduite pour un nombre de gouttes 12 supérieur à 10 et par exemple égal à 14. La monodispersité des gouttes 12 est vérifiée pour qu'elle soit inférieure à 10 %.

Une variante du procédé de formation des gouttes 12 de la dispersion 10 peut être mise en oeuvre dans un appareil 130 illustré sur les Figures 7 et 8.

Comme illustré par ces Figures, l'appareil 130 comporte au moins une buse 32 de formation des gouttes 12 et un réceptacle 33 de réception des gouttes 12 formées.

A la différence de l'appareil 30 représenté sur les Figures 3 et 4, le conduit interne 34 d'amenée de fluide interne 36 débouche dans un conduit intermédiaire 132 d'amenée d'un fluide intermédiaire 134. Le fluide intermédiaire 134 est miscible avec le fluide interne 36 comprenant la première phase 14. Le conduit intermédiaire 132 débouche dans le conduit externe 38.

Le conduit externe 38 débouche dans le réceptacle 33 en étant avantageusement immergé dans le volume de fluide 35.

Dans l'exemple représenté sur la Figure 7, le conduit externe 38 est coudé et présente sensiblement une forme de crosse débouchant dans le réceptacle 33.

Dans cet exemple, le conduit interne 34 et le conduit intermédiaire 132 débouchent vers le haut dans un tronçon amont vertical du conduit externe 38. Le conduit externe 38 débouche vers le bas dans le réceptacle 33.

En plus des moyens 46 d'amenée de fluide interne dans le conduit interne 36 et en des moyens 48 d'amenée de fluide externe dans le conduit externe 38, l'appareil 130 comporte des moyens 136 d'amenée de fluide intermédiaire 134 dans le conduit intermédiaire 132.

Avantageusement, l'appareil 130 comporte une pluralité de buses 32 parallèles raccordées à des moyens communs d'alimentation 46, 48, 136. Un tel appareil est décrit par exemple dans la demande française n°11 55455 de la Demanderesse.

Dans cet exemple, la première phase 14 est huileuse. Elle est par exemple formée à base d'une huile telle que décrite plus haut. Elle comporte avantageusement des molécules d'intérêt cosmétique, tels que des actifs cosmétiques.

Le fluide intermédiaire 134 est miscible avec le fluide interne 36. Elle est par exemple composée d'une phase huileuse identique à la première phase 14 ou miscible avec la première phase 14.

Le fluide intermédiaire 134 est par exemple composé d'au moins une huile choisie parmi le groupe comprenant les huiles de silicone, les huiles minérales, les huiles végétales, les esters d'acide gras et/ou d'alcool gras, typiquement en C1 à C20, et les huiles compatibles avec les esters tels que les solvants apolaires.

Comme on le verra plus bas, le fluide intermédiaire 134 est destiné à former une pellicule 138 autour de la goutte 12 formée à la sortie du conduit intermédiaire 132 dans le fluide externe 40. Ainsi, le fluide intermédiaire 134 retarde la diffusion du premier polymère précurseur du coacervat présent dans le fluide interne 36 jusqu'à ce que le fluide intermédiaire 138 se soit mélangé avec le fluide interne 36.

Dans cet exemple, le premier polymère précurseur du coacervat présent dans le premier fluide 36 est lipophile. Il est avantageusement de type cationique et lipophile. Il peut être formé par exemple d'un polymère dérivé de la silicone tel que l'amodiméticone.

Le fluide externe 40 est aqueux. Le deuxième polymère précurseur du coacervat introduit dans le fluide externe 40 est hydrophile. Il est avantageusement de type anionique. Il est par exemple formé par un acide polyacrylique.

Avantageusement, la phase interne 36 comporte entre 0,05 % en masse et 10 % en masse du premier polymère précurseur du coacervat, par rapport à la masse totale du fluide intermédiaire 36.

De même, le fluide externe 40 comporte entre 0,05 % en masse et 10 % en masse du deuxième polymère précurseur du coacervat, par rapport à la masse totale du fluide externe 40.

Un procédé de formation d'une dispersion 10 de gouttes 12 mis en oeuvre dans l'appareil 130 va maintenant être décrit.

Initialement, le fluide interne 36 est préparé en mélangeant la première phase 14 destinée à former le coeur 17 de la goutte 12 et le premier polymère précurseur du coacervat.

Parallèlement, le fluide externe 40 est préparé en mélangeant un solvant destiné à former la deuxième phase 16 et un deuxième polymère précurseur du coacervat.

Puis, le fluide interne 36, le fluide externe 40 et le fluide intermédiaire 136 sont placés dans les moyens d'amenée 46, 136, 48 respectifs. Ensuite, les moyens d'amenée 46, 48 et 136 sont activés.

Le flux de fluide interne 36 circulant dans le conduit interne 34 débouche dans le conduit intermédiaire 132 pour être entouré par une pellicule 138 de fluide intermédiaire 134. Puis, à l'extrémité du conduit intermédiaire 132, des gouttes 12 de fluide interne 36 totalement entourées par une pellicule 138 de fluide intermédiaire 134 sont formées. Les gouttes 12 circulent alors dans le fluide externe 40 au sein du conduit externe 38.

La pellicule 138 retarde au moins partiellement la diffusion du premier polymère précurseur du coacervat vers l'interface entre la goutte 12 et le fluide externe 40.

Puis, le fluide intermédiaire 134 se mélange progressivement avec le fluide interne 36, permettant au premier polymère précurseur du coacervat de migrer jusqu'à l'interface entre la goutte 12 et le fluide externe 40.
Le deuxième polymère présent dans le fluide externe 40 migre également à cet interface.
La coacervation entre le premier polymère et le deuxième polymère se produit alors à l'interface pour former l'écorce 18. L'écorce 18 formée à cet interface est donc complète, très mince, et n'engendre pas de gélification totale du coeur 17.
Les gouttes 12 ainsi formées sont donc très stables, peu ou pas élastiques, et n'ont pas tendance à coalescer les unes avec les autres. Les gouttes 12 remontent alors dans le réceptacle 33 vers la surface et entrent en contact les unes avec les autres, sans coalescence au moins pendant trois mois à température ambiante et à 50°C.
Lorsque le deuxième polymère est de l'acide acrylique, il est avantageusement maintenu sous forme acide, pour maintenir la viscosité de la phase externe 16 basse, notamment inférieure à 500 mPa.s.
Ceci autorise le crémage des gouttes 12 vers le haut du réceptacle 33.
Dans une variante, la composition de la deuxième phase 16 est modifiée, en ajoutant une solution miscible avec cette phase 16 ou en remplacement partiel de la phase 16.
Un exemple de mise en oeuvre de ce procédé va maintenant être décrit.

<u>Exemple 4 :</u>

Sur le dispositif expérimental des Figures 6 et 7, on met en oeuvre une étape de rigidification entre un premier polymère précurseur de coacervat, de type cationique et lipophile (amodiméticone), et un deuxième polymère précurseur de coacervat de type anionique et hydrophile (acide polyacrylique).
Le premier polymère est contenu dans le fluide interne 36 huileux. Le deuxième polymère est contenu dans le fluide externe 40 aqueuse.
L'étape de rigidification est basée sur la formation d'un coacervat au niveau de l'interface entre l'acide polyacrylique contenu dans le fluide externe 40 et un aminosilicone (amodiméticone) contenu dans le fluide interne 36, après formation de gouttes 12 dans le fluide externe 40.
La rencontre de ces deux polymères provoque la coacervation et la rigidification de la membrane autour des gouttes 12. Dans l'appareil 130 décrit sur la Figure 7 on applique les débits suivants :

Fluide externe : 80 mL/heure ;

Fluide interne : 15 mL/heure ;

Fluide intermédiaire : 5 mL/heure.

La composition de chaque fluide est décrite dans le tableau ci-dessous.

| Phase | Nom du produit | % massique | Fonction |
|---|---|---|---|
| Fluide interne | PDMS 6 sCt | 99,5 % | Solvant |
| | Amino-silicone | 0,5% | Agent de coacervation |
| Fluide externe | Eau | 99,5% | Solvant |
| | Acide polyacrylique | 0,5% | Agent de coacervation |
| Fluide intermédiaire | PDMS 6 sCt | 100 % | solvant |

On obtient, à la suite de la collecte, un réceptacle 33 contenant des gouttes d'environ 1 millimètre de diamètre avec une membrane en coacervat.
Ces gouttes présentent une déformation avant la rupture caractérisée par le coefficient gamma précité égal à 222,37 % avec un coefficient de variance de 9,49 %.
La dispersion 10 ainsi formée comprend 20% en volume de gouttes 12.
Dans une variante, les dispersions 10 exposées précédemment en regard des Figures 1 et 7 contiennent un agent parfumant.
Parmi les agents parfumants, on peut notamment citer tout type de parfum ou de fragrance, ces termes étant utilisés ici de façon indifférente. Ces parfums ou fragrances sont bien connus de l'homme du métier et incluent notamment ceux mentionnés, par exemple, dans S. Arctander, Perfume and Flavor Chemicals (Montclair, N.J., 1969), S. Arctander, Perfume and Flavor Materials of Natural Origin (Elizabeth, N.J., 1960) et dans "Flavor and Fragrance Materials", 1991

(Allured Publishing Co. Wheaton, III. USA).

Les parfums utilisés dans le cadre de la présente invention peuvent comprendre les produits naturels comme les extraits, les huiles essentielles, les absolus, les résinoïdes, les résines, les concrètes, etc... ainsi que les substances basiques de synthèse comme les hydrocarbures, les alcools, les aldéhydes, les cétones, les éthers, les acides, les esters, les acétals, les cétals, les nitriles, etc..., y compris les composés, saturés et insaturés, les composés aliphatiques, alicycliques et hétérocycliques.

Avantageusement, la dispersion 10 selon l'invention est dépourvue d'alcool.

Dans le cadre de la présente description, on entend par « alcool » un composé hydrocarboné, linéaire ou ramifié, comprenant de 1 à 4 atomes de carbone, dans lequel au moins un atome d'hydrogène est remplacé par une fonction hydroxyle. Un tel alcool est typiquement l'éthanol, l'isopropanol, le n-butanol ou tout autre alcool usuel dans le domaine de la cosmétique.

Avantageusement, la première phase 14 et la deuxième phase 16 selon l'invention comprennent une quantité réduite de tensio-actif, généralement tolérée dans le cadre d'une application cosmétique. De préférence, la dispersion 10 est totalement exempte de tensio-actif.

Les gouttes 12 sont formées par un procédé tel que décrit plus haut.

Lorsque la deuxième phase 16 est huileuse et lorsque la première phase 14 est aqueuse, le procédé est mis en oeuvre en disposant l'agent parfumant dans un fluide destiné à former la deuxième phase 16, avantageusement dans le fluide externe 40 destiné à recevoir les gouttes 12 de fluide interne 36.

En variante, les gouttes 12 de la dispersion 10 selon l'invention sont formées dans une deuxième phase 16 huileuse dépourvue d'agent parfumant. Puis, une partie de la deuxième phase 16 huileuse est extraite hors du réceptacle de collecte de la dispersion 10. Cette partie extraite est remplacée par un agent parfumant. La dispersion comporte alors plus de 30 % en masse, avantageusement plus de 35 % en masse de gouttes 12 et plus de 10 % en masse, avantageusement plus de 30 % en masse d'agent parfumant par rapport à la masse totale de la dispersion 10.

Exemple 5 :

Cet exemple met en oeuvre la fabrication d'une dispersion contenant un agent parfumant. L'étape de rigidification est basée sur la formation d'un coacervat au niveau de l'interface entre l'acide polyacrylique contenu dans le fluide interne 36 et un amino silicone contenu dans le fluide réactionnel 44 après formation des gouttes 12. Les débits suivants sont appliqués :

Fluide interne : 40 mL/heure ;
Fluide externe : 100 mL/heure ;
Fluide réactionnel : 10 mL/heure.

La composition de chaque fluide est décrite dans le tableau ci-dessous :

| Phase | Nom du produit | % massique | Fonction |
|---|---|---|---|
| Fluide interne | Eau | 99,5% | Solvant |
| | Acide polyacrylique | 0,5% | Agent de coacervation |
| Fluide externe | Isononyl Isononoate | 33% | Solvant |
| | Parfum | 66% | Parfum |
| Fluide réactionnel | Diethylethanolamine | 0.5% | Base |
| | Amino-silicone | 1% | Agent de coacervation |
| | Isononyl Isononoate | 98.5% | Solvant |

Ce procédé permet d'obtenir une dispersion 10 de gouttes 12 monodispersées de diamètre égal à 1 mm.

Dans une variante, la dispersion 10 comporte des gouttes 12 d'une première phase 14 huileuse dispersée dans une deuxième phase 16 aqueuse. L'agent parfumant est disposé dans les gouttes 12 de première phase 14. Dans ce cas, le fluide interne 36 est huileux et contient l'agent parfumant, en plus du premier polymère précurseur de la coacervation. La phase intermédiaire 134 est miscible avec le fluide interne 36.

Le fluide externe 40 est aqueux. Il contient le deuxième polymère précurseur de la coacervation. Eventuellement, d'autres molécules, notamment des actifs cosmétiques, peuvent être ajoutées dans le fluide externe 40.

La teneur massique en agent parfumant dans le fluide interne 36, par rapport à la masse totale du fluide interne

36, est supérieure à 40 % en masse et est notamment comprise entre 50 % en masse et 70 % en masse.

Des exemples de fabrication de dispersions 10 contenant des gouttes 12 de phase huileuse, munies d'un agent parfumant, dispersées dans une deuxième phase 16 aqueuse vont maintenant être décrits.

Exemple 6 :

Cet exemple met en oeuvre la fabrication d'une dispersion contenant un agent parfumant. Cette fabrication est effectuée dans un appareil 130 tel que représenté sur les Figures 7 et 8.

L'étape de rigidification est basée sur la formation d'un coacervat au niveau de l'interface entre l'acide polyacrylique contenu dans le fluide externe 40, et un aminosilicone contenu dans le fluide interne 36 après formation des gouttes, et après mélange entre le fluide intermédiaire 134 de la pellicule 138 et le fluide interne 36. Sur l'appareil 130 décrit, on applique les débits suivants :

- fluide interne : 15 mL/heure ;
- fluide externe : 80 mL/heure ;
- fluide intermédiaire : 5 mL/heure.

La composition de chaque fluide est décrite dans le tableau ci-dessous :

| Phase | Nom du produit | % massique | Fonction |
|---|---|---|---|
| Fluide interne | Isononyl Isononoate | 33 % | Solvant |
| | Parfum | 66% | |
| | Amino-silicone | 1 % | Agent de coacervation |
| Fluide externe | Eau | 99,5% | Solvant |
| | Acide polyacrylique | 0,5% | Agent de coacervation |
| Fluide intermédiaire | Isononyl Isononoate | 100 % | solvant |

On obtient ainsi une dispersion 10 de gouttes 12 de première phase 14 huileuse contenant un agent parfumant, dispersées dans une deuxième phase 14 aqueuse. Les gouttes 12 sont monodispersées, présentent un diamètre de 1 millimètre et une écorce 18 en coacervat.

La teneur massique en gouttes dans la dispersion 10 est de 20% en masse, et la teneur massique en parfum dans la dispersion est de10 % en masse, par rapport à la masse totale de la dispersion.

Exemple 7 :

Un procédé analogue à celui de l'exemple 6 est mis en oeuvre. Toutefois, à la différence du procédé décrit dans l'exemple 6, une partie de la deuxième phase 16 obtenue à l'issue de la production de gouttes 12 (par exemple au bout d'une heure) est éliminée et est remplacée par une solution de triéthanolamine à 0,4%. Cette solution est apte à gélifier l'acide polyacrylique présent dans la phase aqueuse. La dispersion comporte alors 40 % en masse de gouttes 12 et 20 % en masse d'agent parfumant.

Exemple 8 :

Cet exemple est identique à l'exemple 7, à la différence que l'appareil 130 est configuré pour former des gouttes 12 de 2 millimètres de diamètre. La dispersion finale 10 comporte alors 40 % en masse de gouttes de 2 millimètres de diamètre et 20 % en masse de parfum.

Exemple 9 :

Cet exemple est identique à l'exemple 6, à la différence qu'à l'issue de la production de gouttes 12 (par exemple au bout d'une heure), une partie de la deuxième phase 16 est éliminée et est remplacée par une composition cosmétique décrite dans le tableau ci-dessous :

| Phase | Nom du produit | % massique |
|---|---|---|
| A | Eau | 87,050% |
| A | Acide polyacrylique | 0,150% |
| B | Glycerine | 5,000% |
| B | Propylène glycol | 5,000% |
| B | Gomme de xanthane | 0,250% |
| C | Eau | 2,000% |
| C | Chlorphenesin (3-(4-chlorophenoxy)-1,2 - propanediol) | 0,300% |
| C | Phenoxyethanol | 0,250% |

Les phases A, B et C sont préparées séparément. La phase B est ensuite ajoutée à la phase A, puis une fois la solution homogène, la phase C est ajoutée en mélange.

Une fois la composition cosmétique mélangée avec la dispersion, une solution de triéthanolamine à 1,3 % (10mL) est ajoutée dans la deuxième phase 16 afin de gélifier l'acide polyacrylique. Cette dispersion comporte alors 10 % en masse de gouttes et 5 % en masse de parfum.

Exemple 10 :

Cet exemple est identique à l'exemple 6. Toutefois, à la différence de l'exemple 6, à l'issue de la production de gouttes 12 (par exemple au bout d'une heure), une partie de la deuxième phase 16 est éliminée et est remplacée par de l'eau. Ceci diminue la concentration en acide polyacrylique dans la deuxième phase 16. Après homogénéisation, une partie de la deuxième phase 16 est à nouveau éliminée et solution de triéthanolamine à 0,23 % est ajoutée. La dispersion obtenue comporte alors 20 % en masse de gouttes 12 et 10 % en masse de parfum.

La teneur massique finale en acide polyacrylique dans la deuxième phase 16 est inférieure à 1 %, et est notamment de 0,073 % dans la deuxième phase 16. Ceci conduit à une viscosité acceptable pour que la dispersion 10 soit pulvérisable.

Plus généralement, grâce à l'invention qui vient d'être décrite, il est possible d'obtenir une composition cosmétique parfumante comprenant la dispersion selon l'invention. Cette composition cosmétique parfumante présente les avantages suivants.

En premier lieu, il n'est pas nécessaire d'utiliser un alcool, notamment de l'éthanol, pour préparer la dispersion 10 selon l'invention. En effet, la dispersion 10 étant biphasée, il est possible de disperser l'agent parfumant dans une faible quantité de phase huileuse.

La dispersion selon l'invention est particulièrement stable. Les gouttes 12 munies d'une écorce 18 de coacervat bloquent les phénomènes de coalescence, ce qui permet de maintenir l'intégrité de la dispersion 10.

La dispersion 10 est en outre facilement pulvérisable. La viscosité des phases 14, 16 est suffisamment faible pour permettre une telle pulvérisation et l'écorce 18 de coacervat présente une résistance mécanique telle qu'elle est détruite par le passage dans un dispositif de pulvérisation.

En outre, les gouttes 12 sont non collantes, comme exposé plus haut. La formation de l'écorce 18 de coacervat ne nécessite donc pas l'utilisation d'un tensio-actif qui produit dans certains cas un effet collant dans les formulations de parfums sans alcool de type nano ou micro-émulsion.

La dispersion 10 selon l'invention peut comprendre des taux de charge en agent parfumant élevés, par exemple supérieurs à 10 % en masse, notamment supérieurs à 20% en masse et pouvant atteindre 40 % en masse. Ceci est facilement atteignable, et en l'absence d'alcool dans la dispersion 10.

Dans une variante, des agents modificateurs de profil olfactif, par exemple d'augmentation de la rémanence du parfum, sont ajoutés dans la première phase 14 et/ou dans la deuxième phase 16.

Ces agents modificateurs de profil olfactif sont par exemple des copolymères lipophiles des esters, ou des alcools de guerbet.

Dans une variante, une solution de xanthane est ajoutée dans la deuxième phase 16 pour modifier les propriétés du gel final, après gélification de la phase 16.

Dans encore une autre variante, il est possible de récupérer les gouttes 12 de première phase 14 et de les redisperser dans une nouvelle deuxième phase 16.

## Revendications

1. Procédé de formation de gouttes (12) d'une première phase (14) dispersées dans une deuxième phase (16) sensiblement immiscible avec la première phase (14), la solubilité de la première phase (14) dans la deuxième phase (16) étant inférieure à 5 % en masse, chaque goutte (12) comportant un coeur (17) formé de première phase (14) et une écorce (18) formée d'une couche de coacervat interposée entre la première phase (14) et la deuxième phase, le procédé comprenant l'étape suivante :

   - fourniture d'un premier fluide (36) comprenant la première phase (14) et un premier polymère précurseur du coacervat contenu dans la première phase (14) ;

   **caractérisé en ce qu'**il comprend les étapes suivantes :

   - formation de gouttes de premier fluide (36) dans un deuxième fluide (40) destiné à former la deuxième phase (16) ;
   - amenée d'un deuxième polymère précurseur du coacervat dans le deuxième fluide (40) ;
   - lors de la formation de chaque goutte (12), ou une fois chaque goutte (12) formée, création de la couche de coacervat par interaction entre le premier polymère précurseur et le deuxième polymère précurseur à l'interface entre la première phase (14) et la deuxième phase (16),

   l'une de la première phase (14) et de la deuxième phase (16) étant aqueuse et l'autre de la première phase (14) et de la deuxième phase (16) étant huileuse.

2. Procédé selon la revendication 1, **caractérisé en ce que** l'étape de formation de gouttes (12) comprend la formation de gouttes de premier fluide (36) à la sortie d'un premier conduit (34) débouchant dans le deuxième fluide (40).

3. Procédé selon la revendication 2, **caractérisé en ce que** la deuxième phase (16) est mise en circulation dans un deuxième conduit (38), la sortie du premier conduit (34) débouchant dans le deuxième conduit, avantageusement coaxialement avec l'axe local du deuxième conduit (38).

4. Procédé selon l'une quelconque des revendications précédentes, **caractérisé en ce que** les gouttes (12) de première phase (14) présentent un diamètre supérieur à 500 microns, avantageusement inférieur à 3000 microns, notamment compris entre 800 microns et 2000 microns.

5. Procédé selon l'une quelconque des revendications précédentes, **caractérisé en ce que** l'amenée du deuxième polymère précurseur dans le deuxième fluide (40) est effectuée après la formation de la ou de chaque goutte (12), avantageusement dans un fluide réactionnel miscible avec le deuxième fluide (40).

6. Procédé selon l'une quelconque des revendications précédentes, **caractérisé en ce qu'**il comporte la formation d'une pellicule (138) d'un fluide intermédiaire (134) miscible avec le premier fluide (36) autour de chaque goutte (12) de premier fluide (36) dans le deuxième fluide (40), puis le mélange entre la pellicule (138) de fluide intermédiaire (134) et le premier fluide (36) pour mettre en contact le premier polymère précurseur du coacervat avec le deuxième polymère précurseur du coacervat.

7. Procédé selon l'une quelconque des revendications précédentes, **caractérisé en ce qu'**il comporte l'amenée dans le deuxième fluide (40) d'un agent réactionnel propre à engendrer l'interaction entre le premier polymère précurseur et le deuxième polymère précurseur pour former l'écorce (18) après la formation de chaque goutte (12).

8. Procédé selon l'une quelconque des revendications précédentes, **caractérisé en ce que** l'interaction entre le premier polymère précurseur du coacervat et le deuxième polymère précurseur du coacervat pour former l'écorce (18) est une interaction ionique.

9. Procédé selon la revendication 8, **caractérisé en ce que** le premier précurseur du coacervat est un premier polymère susceptible d'être ionisé pour présenter une première charge, le deuxième précurseur du coacervat étant un deuxième polymère susceptible d'être ionisé pour présenter une deuxième charge opposée à la première charge.

10. Procédé selon la revendication 9, **caractérisé en ce que** l'un du premier précurseur et du deuxième précurseur est un polymère lipophile susceptible d'être ionisé au contact d'une phase aqueuse, par exemple un polymère

contenant une silicone et contenant un groupe fonctionnel susceptible d'être ionisé, le polymère lipophile étant avantageusement un dérivé de la dimethicone, comme l'amodimethicone et ses dérivés.

**11.** Procédé selon la revendication 10 **caractérisé en ce que** le polymère lipophile est un polymère lipophile cationique tel qu'un polymère présentant une fonction amine primaire, secondaire ou tertiaire, ou tel qu'un polymère silicone présentant des fonctions amines, ou encore est un polymère lipophile anionique, comme un polymère présentant une fonction acide carboxylique tel que l'acide polyacrylique, ou présentant une fonction acide acrylique, une fonction acide métacrylique ou une fonction acide maléique.

**12.** Procédé selon l'une quelconque des revendications 9 à 11, **caractérisé en ce que** l'autre du premier polymère précurseur et du deuxième polymère précurseur est un polymère hydrophile susceptible d'être ionisé.

**13.** Procédé selon la revendication 12, **caractérisé en ce que** l'autre du premier précurseur et du deuxième précurseur est un polymère hydrophile acrylique tel qu'un copolymères d'acide acrylique ou d'acide maléique et d'au moins un autre monomère, tels que l'acrylamide, les acrylates d'alkyle, les acrylates d'alkyle en $C_5$-$C_8$, les acrylates d'alkyle en $C_{10}$-$C_{30}$, les méthacrylates d'alkyle en $C_{12}$-$C_{22}$, les méthacrylates méthoxypolyéthylèneglycol, les acrylates d'hydroxyester, ou un polymère hydrophile cationique tel que le chitosan ou les dérivés de gomme guar, notamment le chlorure d'hydroxypropyltrimonium guar.

## Patentansprüche

**1.** Verfahren zum Bilden von Tropfen (12) einer ersten Phase (14) dispergiert in einer zweiten Phase (16), die im Wesentlichen unmischbar mit der ersten Phase (14) ist, wobei die Löslichkeit der ersten Phase (14) in der zweiten Phase (16) kleiner als 5 Massenprozent ist, wobei jeder Tropfen (12) einen Kern (17), welcher aus der ersten Phase (14) gebildet ist, und eine Schale (18) umfasst, welche aus einer Koazervatschicht gebildet ist, angeordnet zwischen der ersten Phase (14) und der zweiten Phase,
wobei das Verfahren den folgenden Schritt umfasst:

- Bereitstellen eines ersten Fluids (36) umfassend die erste Phase (14) und ein in der ersten Phase (14) enthaltenes erstes Koazervatvorläuferpolymer; **gekennzeichnet dadurch, dass** es die folgenden Schritte umfasst:

  - Bilden von Tropfen des ersten Fluids (36) in einem zweiten Fluid (40), welches bestimmt ist, die zweite Phase (16) zu bilden;
  - Einbringen eines zweiten Koazervatvorläuferpolymers in das zweite Fluid (40);
  - Erzeugen der Koazervatschicht durch Wechselwirkung zwischen dem ersten Vorläuferpolymer und dem zweiten Vorläuferpolymer an der Grenzfläche zwischen der ersten Phase (14) und der zweiten Phase (16), während der Bildung jedes Tropfens (12) oder sobald sich jeder Tropfen (12) gebildet hat,

wobei eine von der ersten Phase (14) und der zweiten Phase (16) wässrig ist und die andere von der ersten Phase (14) und der zweiten Phase (16) ölig ist.

**2.** Verfahren gemäß Anspruch 1, **gekennzeichnet dadurch, dass** der Schritt des Bildens von Tropfen (12) die Bildung von Tropfen des ersten Fluids (36) am Auslass einer ersten Leitung (34), die in das zweite Fluid mündet, umfasst.

**3.** Verfahren gemäß Anspruch 2, **gekennzeichnet dadurch, dass** die zweite Phase (16) in einer zweiten Leitung (38) zirkuliert wird, wobei der Auslass der ersten Leitung (34) in die zweite Leitung mündet, vorteilhaft koaxial zur lokalen Achse der zweiten Leitung (38).

**4.** Verfahren gemäß einem der voranstehenden Ansprüche, **gekennzeichnet dadurch, dass** die Tropfen (12) der ersten Phase (14) einen Durchmesser von größer als 500 Mikrometer, vorteilhaft kleiner als 3000 Mikrometer, insbesondere zwischen 800 Mikrometer und 2000 Mikrometer aufweisen.

**5.** Verfahren gemäß einem der voranstehenden Ansprüche, **gekennzeichnet dadurch, dass** das Einbringen des zweiten Vorläuferpolymers in das zweite Fluid (40) nach der Bildung des oder jedes Tropfens (12) erfolgt, vorteilhaft in einem Reaktionsfluid, das mit dem zweiten Fluid (40) mischbar ist.

**6.** Verfahren gemäß einem der voranstehenden Ansprüche, **gekennzeichnet dadurch, dass** es den Schritt des Bildens eines Films (138) eines Zwischenfluids (134), das mit dem ersten Fluid (36) mischbar ist, um jeden Tropfen (12) des ersten Fluids (36) in dem zweiten Fluid (40) herum, und anschließend Mischen des Films (138) des Zwischenfluids (134) und des ersten Fluids (36) umfasst, um das erste Koazervatvorläuferpolymer mit dem zweiten Koazervatvorläuferpolymer in Kontakt zu bringen.

7. Verfahren gemäß einem der voranstehenden Ansprüche, **gekennzeichnet dadurch, dass** es das Einbringen eines Reaktionsmittels in das zweite Fluid (40) umfasst, welches geeignet ist, das Wechselwirken zwischen dem ersten Vorläuferpolymer und dem zweiten Vorläuferpolymer zum Bilden der Schale (18) nach der Bildung eines jeden Tropfens (12) herbeizuführen.

8. Verfahren gemäß einem der voranstehenden Ansprüche, **gekennzeichnet dadurch, dass** das Wechselwirken zwischen dem ersten Koazervatvorläuferpolymer und dem zweiten Koazervatvorläuferpolymer zum Bilden der Schale (18) eine ionische Wechselwirkung ist.

9. Verfahren gemäß Anspruch 8, **gekennzeichnet dadurch, dass** das erste Koazervatvorläuferpolymer ein erstes Polymer ist, welches ionisiert werden kann, um eine erste Ladung aufzuweisen, und das zweite Koazervatvorläuferpolymer ein zweites Polymer ist, welches ionisiert werden kann, um eine zweite Ladung, gegensätzlich zur ersten Ladung, aufzuweisen.

10. Verfahren gemäß Anspruch 9, **gekennzeichnet dadurch, dass** einer von dem ersten Vorläufer und dem zweiten Vorläufer ein lipophiles Polymer ist, welches in Kontakt mit einer wässrigen Phase ionisiert werden kann, beispielweise ein Polymer enthaltend ein Silikon und enthaltend eine funktionelle Gruppe, die ionisiert werden kann, wobei das lipophile Polymer vorteilhaft ein Derivat von Dimethicone ist, wie Amodimethicone und seine Derivate.

11. Verfahren gemäß Anspruch 10, **gekennzeichnet dadurch, dass** das lipophile Polymer ein kationisches lipophiles Polymer ist, wie ein Polymer mit einer primären, sekundären oder tertiären Aminfunktionalität, oder wie ein Silikonpolymer mit Aminfunktionalitäten, oder aber ein anionisches lipophiles Polymer ist, wie ein Polymer mit einer Carbonsäurefunktionalität, wie Polyacrylsäure, oder mit einer Acrylsäurefunktionalität, einer Methacrylsäurefunktionalität oder einer Maleinsäurefunktionalität.

12. Verfahren gemäß einem der Ansprüche 9 bis 11, **gekennzeichnet dadurch, dass** das andere von dem ersten Vorläuferpolymer und dem zweiten Vorläuferpolymer ein hydrophiles Polymer ist, welches ionisiert werden kann.

13. Verfahren gemäß Anspruch 12, **gekennzeichnet dadurch, dass** der andere von dem ersten Vorläufer und dem zweiten Vorläufer ein hydrophiles Acrylpolymer ist, wie Copolymere von Acrylsäure oder Maleinsäure und von mindestens einem anderen Monomer, wie Acrylamide, Alkylacrylate, $C_5$-$C_8$ Alkylacrylate, $C_{10}$-$C_{30}$ Alkylacrylate, $C_{12}$-$C_{22}$ Alkylmethacrylate, Methoxypolyethylenglykolmethacrylate, Hydroxyesteracrylate, oder ein kationisches hydrophiles Polymer, wie Chitosan oder Derivate von Guar-Gummi, insbesondere Guarhydroxypropyltrimoniumchlorid.

## Claims

1. Method for forming drops (12) of a first phase (14) which are dispersed in a second phase (16) which is substantially immiscible with the first phase (14), the solubility of the first phase (14) in the second phase (16) being less than 5% by mass, each drop (12) having a core (17) formed of first phase (14) and a shell (18) formed of a coacervate layer interposed between the first phase (14) and the second phase,
the method comprising the following step:

   - providing a first fluid (36) comprising the first phase (14) and a first precursor polymer of the coacervate contained in the first phase (14);

**characterised in that** it comprises the following steps:

   - forming drops of first fluid (36) in a second fluid (40) which is to form the second phase (16);
   - introducing a second precursor polymer of the coacervate into the second fluid (40);
   - during the formation of each drop (12), or once each drop (12) has formed, creating the coacervate layer by interaction between the first precursor polymer and the second precursor polymer at the interface between the

first phase (14) and the second phase (16),

one of the first phase (14) and the second phase (16) being aqueous and the other of the first phase (14) and the second phase (16) being oily.

2. Method according to claim 1, **characterised in that** the step of forming drops (12) comprises the formation of drops of first fluid (36) at the outlet of a first pipe (34) which opens into the second fluid (40).

3. Method according to claim 2, **characterised in that** the second phase (16) is circulated in a second pipe (38), the outlet of the first pipe (34) opening into the second pipe, advantageously coaxially with the local axis of the second pipe (38).

4. Method according to any one of the preceding claims, **characterised in that** the drops (12) of first phase (14) have a diameter greater than 500 microns, advantageously less than 3000 microns, especially between 800 microns and 2000 microns.

5. Method according to any one of the preceding claims, **characterised in that** the introduction of the second precursor polymer into the second fluid (40) is carried out after the formation of the or each drop (12), advantageously in a reaction fluid which is miscible with the second fluid (40).

6. Method according to any one of the preceding claims, **characterised in that** it comprises the formation of a film (138) of an intermediate fluid (134) which is miscible with the first fluid (36) around each drop (12) of first fluid (36) in the second fluid (40), then mixing between the film (138) of intermediate fluid (134) and the first fluid (36) in order to bring the first precursor polymer of the coacervate into contact with the second precursor polymer of the coacervate.

7. Method according to any one of the preceding claims, **characterised in that** it comprises the introduction into the second fluid (40) of a reaction agent which is capable of causing the interaction between the first precursor polymer and the second precursor polymer to form the shell (18) after the formation of each drop (12).

8. Method according to any one of the preceding claims, **characterised in that** the interaction between the first precursor polymer of the coacervate and the second precursor polymer of the coacervate to form the shell (18) is an ionic interaction.

9. Method according to claim 8, **characterised in that** the first precursor of the coacervate is a first polymer capable of being ionised in order to have a first charge, the second precursor of the coacervate being a second polymer capable of being ionised in order to have a second charge opposite to the first charge.

10. Method according to claim 9, **characterised in that** one of the first precursor and the second precursor is a lipophilic polymer capable of being ionised in contact with an aqueous phase, for example a polymer containing a silicone and containing a functional group capable of being ionised, the lipophilic polymer advantageously being a dimethicone derivative, such as amodimethicone and its derivatives.

11. Method according to claim 10, **characterised in that** the lipophilic polymer is a cationic lipophilic polymer such as a polymer having a primary, secondary or tertiary amine functional group, or such as a silicone polymer having amine functional groups, or is an anionic lipophilic polymer, such as a polymer having a carboxylic acid functional group such as polyacrylic acid, or having an acrylic acid functional group, a methacrylic acid functional group or a maleic acid functional group.

12. Method according to any one of claims 9 to 11, **characterised in that** the other of the first precursor polymer and the second precursor polymer is a hydrophilic polymer capable of being ionised.

13. Method according to claim 12, **characterised in that** the other of the first precursor and the second precursor is an acrylic hydrophilic polymer such as a copolymer of acrylic acid or maleic acid and at least one other monomer, such as acrylamide, alkyl acrylates, $C_5$-$C_8$-alkyl acrylates, $C_{10}$-$C_{30}$-alkyl acrylates, $C_{12}$-$C_{22}$-alkyl methacrylates, methoxypolyethylene glycol methacrylates, hydroxyester acrylates, or a cationic hydrophilic polymer such as chitosan or guar gum derivatives, especially guar hydroxypropyltrimonium chloride.

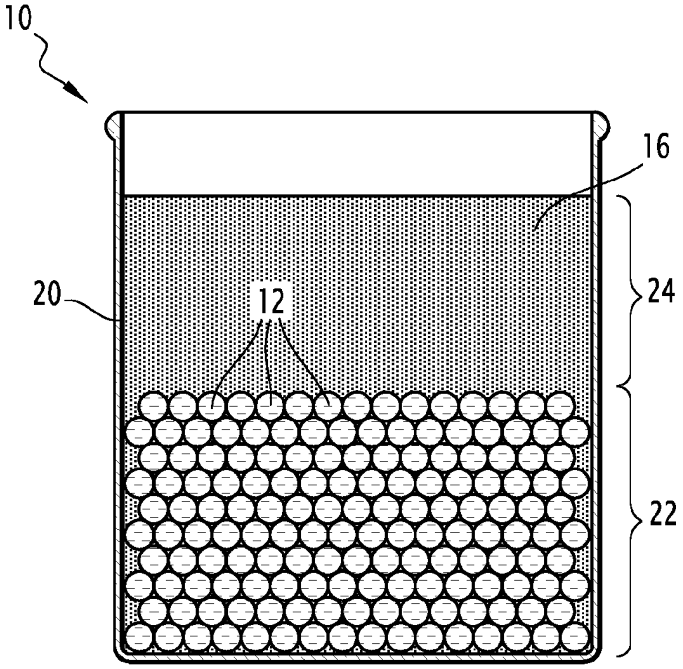


FIG.1

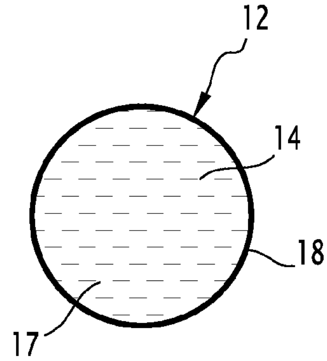


FIG.2

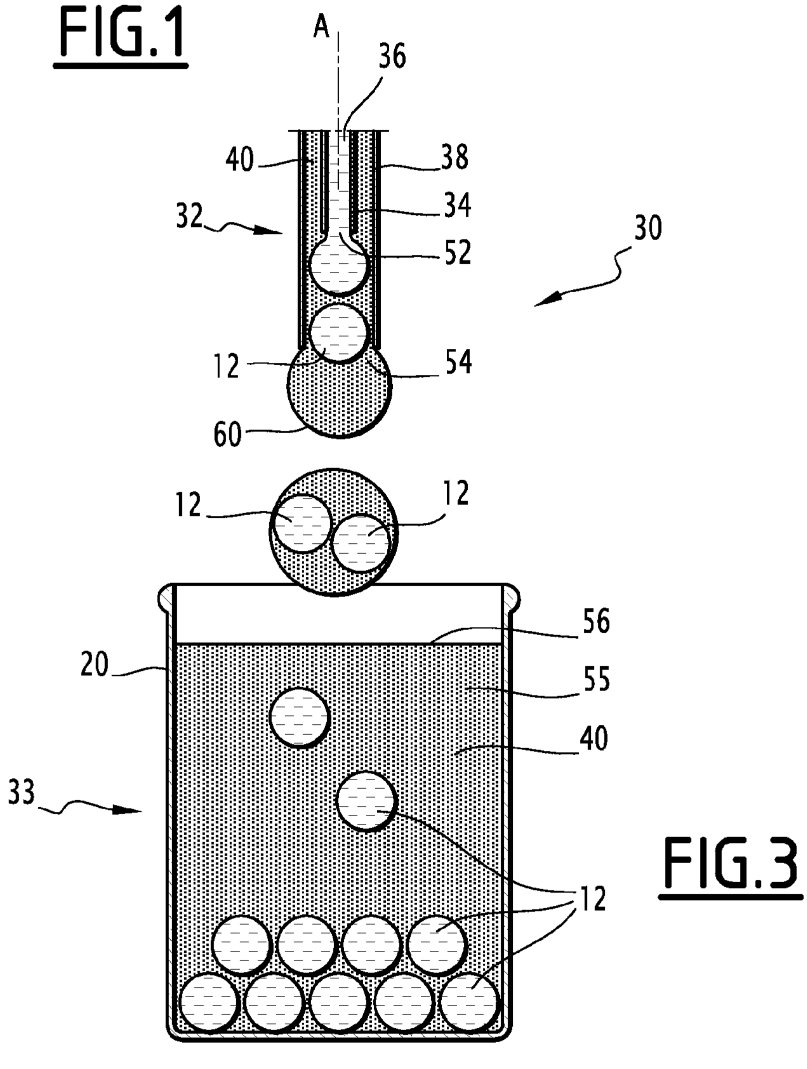


FIG.3

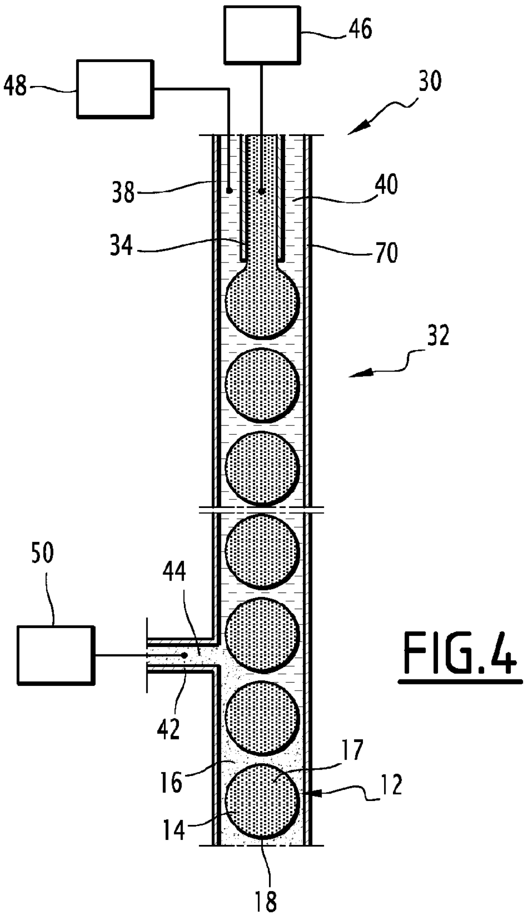
46
48
30
38
40
34
70
32
50
44
42
17
16
12
14
18

FIG.4

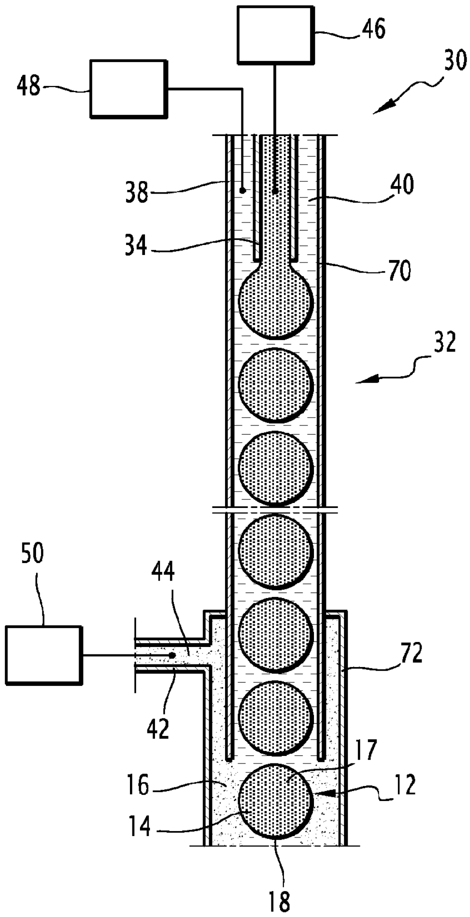
46
48
30
38
40
34
70
32
50
44
72
42
17
16
12
14
18

FIG.5

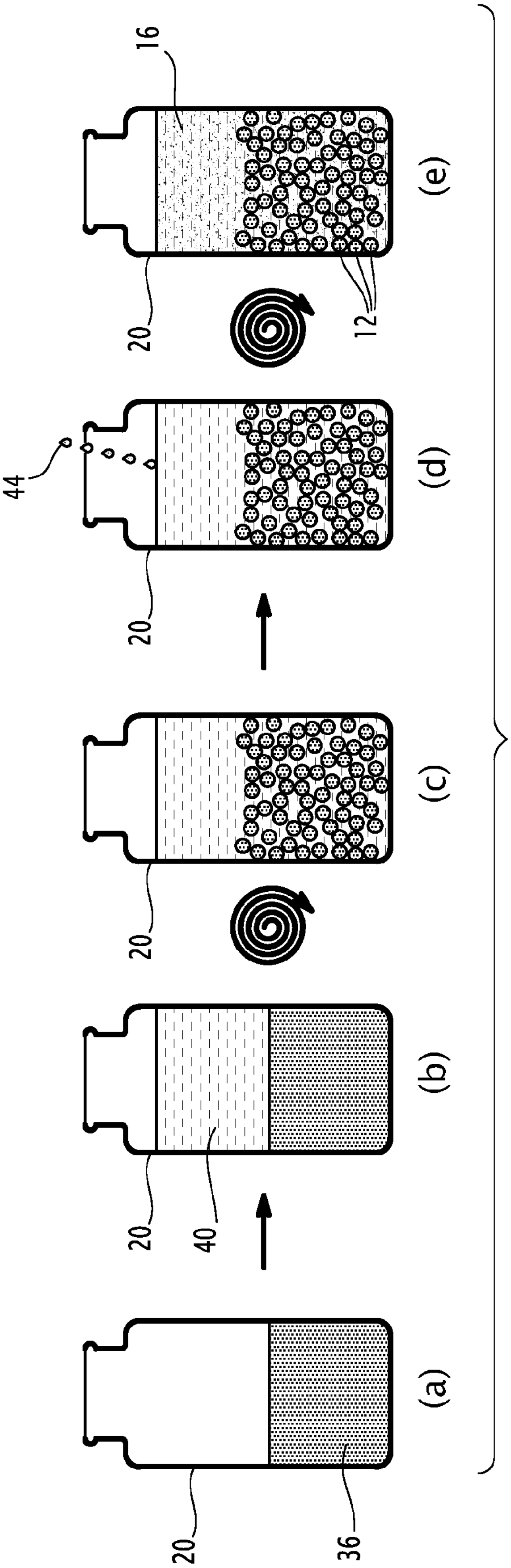


FIG.6

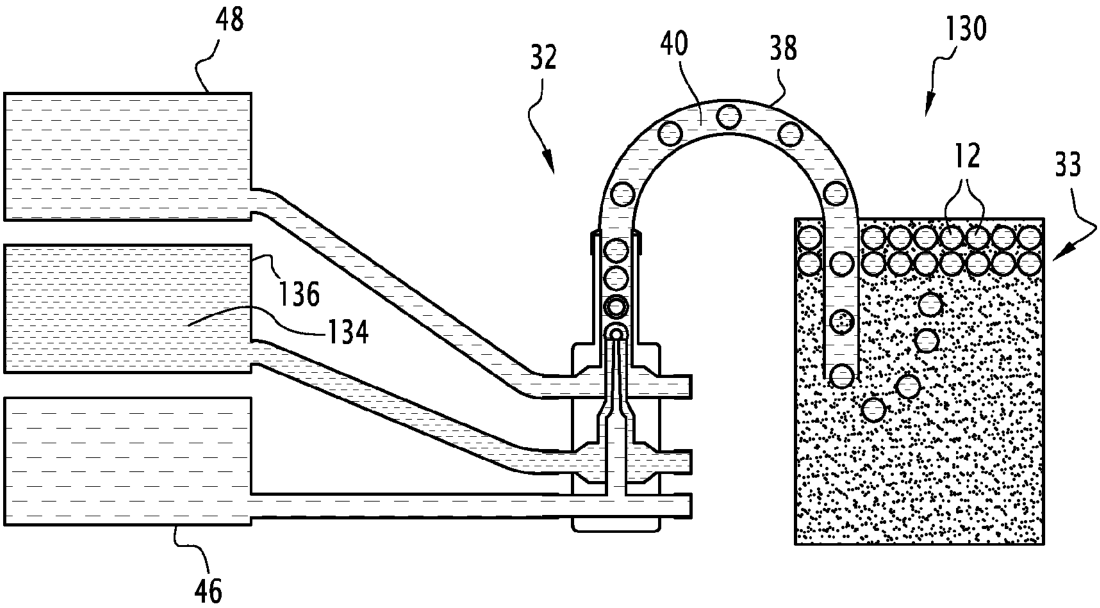
48
32
40
38
130
12
33
136
134
46

FIG.7

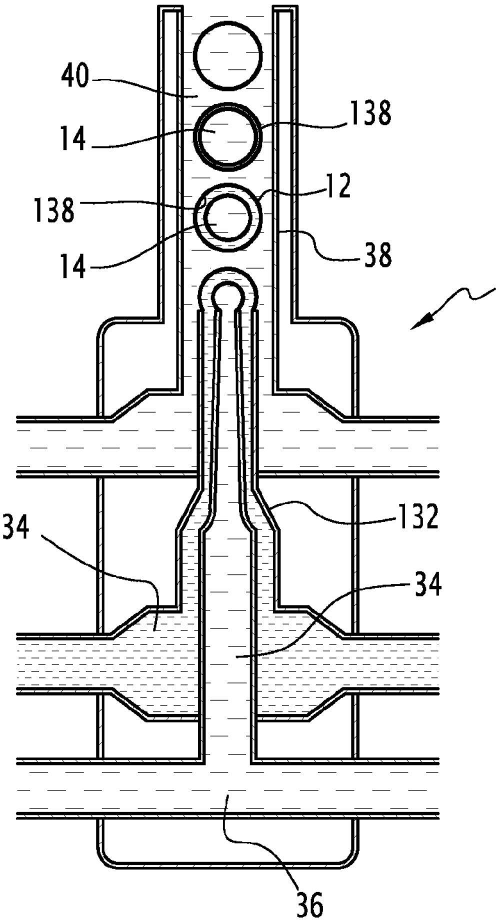
40
14
138
12
138
14
38
32
134
132
34
36

FIG.8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1524030 A1
- WO 03002248 A
- FR 1155455


### Littérature non-brevet citée dans la description


- **MASON et al.** *J. Coll. Int. Sci.,* 1996, vol. 179, 439-448
- **SATO et al.** *J. Chem. Phys.,* 2007, vol. 111, 1393-1401
- **S. ARCTANDER.** *Perfume and Flavor Chemicals,* 1969
- **S. ARCTANDER.** *Perfume and Flavor Materials of Natural Origin,* 1960
- Flavor and Fragrance Materials. Allured Publishing Co, 1991